# EUROPEAN PATENT SPECIFICATION

(11) **EP 3 088 011 B1**
(45) Date of publication and mention of the grant of the patent: **22.08.2018**
(21) Application number: 14873948.5
(22) Date of filing: 23.12.2014
(51) Int. Cl.: A61L 27/16, A61L 27/40, A61L 27/52, A61F 2/08, A61F 2/12, A61F 2/26, C08F 251/00, C08L 33/26, C08L 51/02

(54) **POLYACRYLAMIDE HYDROGEL-BASED MATERIAL FOR MEDICAL PURPOSES AND METHOD FOR PRODUCING SAME**
HYDROGELBASIERTES POLYACRYLAMIDMATERIAL FÜR MEDIZINISCHE ZWECKE UND VERFAHREN ZUR HERSTELLUNG DAVON
MATÉRIAU À VOCATION MÉDICALE À BASE D'HYDROGEL DE POLYACRYLAMIDE ET PROCÉDÉ DE FABRICATION

(30) Priority: 26.12.2013 RU 2013157847
(43) Date of publication of application: 02.11.2016
(73) Proprietor: International Business Company "Newcross Ventures Ltd.", Belize City (BZ)
(72) Inventor: VLASOV, Victor Ivanovich, Moscow 121351 (RU); KUZNETSOVA, Elizaveta Anatolyevna, Moscow 107241 (RU); LOPATIN, Vladislav Victorovich, Moscow 119270 (RU); DARYEVICH, Evgeniy Nikolaevich, Moscow 123022 (RU)
(74) Representative: Müller & Schubert
(86) International application number: PCT/RU2014/000969
(87) International publication number: WO 2015/099578

(56) References cited:
- EP-A1- 2 497 468
- DE-A1-102009 042 037
- RU-C1- 2 127 095
- RU-C1- 2 127 129
- US-A1- 2003 065 389
- JENNIFER A. LEDON ET AL.: 'Inflammatory Nodules Following Soft Tissue Filler Use: A Review of Causative Agents, Pathology and Treatment Options' AM J CLIN DERMATOL vol. 14, no. 5, September 2013, pages 401 - 411, XP008183778

## Description

### FIELD OF THE INVENTION

The invention is related to formulations and methods for manufacture of a biocompatible hydrogel based on a cross-linked copolymer of acrylamide and linking agents, the gel can be used as material for medical purposes, for example:
- as endoprosthetic material for specific injection of hydrogel for the purpose of plastic correction of facial soft tissue, breast tissue, penis, calves, vocal cords and other tissues, the density of which is the same as hydrogel density; as well as in urology and orthopedics, mainly in orthopedics, as synovial fluid endoprosthesis.

### DESCRIPTION OF RELATED ART

The application of polyacrylamide gels in medicinal practice is widely known (see Lopatin V.V. "Polyacrylamide hydrogels in medicine", publisher Scientific world, 2004).

In particular, there is also data on a polyfunctional biocompatible hydrogel (patent RU 2205034 published on May 27, 2003), containing 1.3 - 15.0 mass% of acrylamide, linking agents - N,N'-methylene-bis-acrylamide - 0.004-0.975%, N,N-ethylene-bis-acrylamide - 0.004-5.1%, poviargolum - 0.002-0.45% and water - up to 100%. The hydrogel is made by copolymerization of acrylamide with linking agents in an aqueous medium in the presence of a peroxide polymerization activator, the incubation of reaction mass is carried out in two stages, where the first stage is performed at the temperature of 20-90°C for 2-24 hours, and the second stage is performed at 107-130°C for not more than 2 hours. Hydrogel causes low tissue reaction to its implantation and has reduced possibility of colonization by pathogenic flora.

There is also data on polyfunctional biocompatible hydrogel (patent RU 2236872, published on September 27, 2004), containing in mass% acrylamide - 1.95 - 8.0%, methacrylamide - 0.54 - 3.0%, 2-hydroxyethyl methacrylate - 0.003 - 0.4%, N,N'-methylene-bis-acrylamide - 0.006 - 0.6% and water - up to 100%. This hydrogel is manufactured by copolymerization of the mentioned monomers in the aqueous medium in the presence of a peroxide polymerization activator, the incubation of reaction mass is carried out in three stages: the first stage at a temperature of 20 - 30°C for 12-24 hours, the second stage is γ-irradiation in dose of 0.4-1.0 megarad, and the third stage at a temperature of 100 - 130°C and pressure of 0-1.2 atm for 20 - 40 mins.

Such gels were widely used as endoprosthesis of synovial fluid (see Abu-Zakhra T.M. "Application of artificial synovial fluid based on polyacrylamide gel in treatment of knee joint arthrosis", author's abstract of dissertation 14.00.22 / Abu-Zakhra Tarek Musa-Jaser. - Moscow, 2004.; Dirsh A.V. "Research of interaction of polyacrylamide hydrogels with biological tissues", scientific library of dissertations and author's abstracts disserCat http://www.dissercat.com/content/issledovanie-vzaimodeistviya-poliakrilamidnykh-gidrogelei-s-biologicheskimi-tkanyami#ixzz2K9B1XY18).

However, all acrylamide-based polymers do not resorb in human tissue for a long time. When gels are present in the body for a long period of time, there is a risk of inflammation to occur, which can require additional surgical intervention to remove the gel.

This disadvantage is also related to the most technically similar to this invention the polyfunctional biocompatible hydrogel (patent RU 2127095 published on March 10, 1999), containing in mass% 4.8 - 8.0 mass% of acrylamide copolymer and methylene-bis-acrylamide taken in mass ration 100:0.5-5.0% and water - up to 100%. This hydrogel is obtained by copolymerization of acrylamide with N,N-ethylene-bis-acrylamide in an aqueous medium (pH 9.0 - 9.5) in the presence of a peroxide polymerization activator, the reaction mass is incubated at the temperature of 20 - 90°C for 2-24 hours and then at a temperature of 100 - 105°C (see patent RU 2127129 published March 10, 1999).

DE 10 2009 042037 discloses materials for the production of synthetic extracellular matrices used in implants comprising methacrylated hyaluronic acid and an acrylamide/N,N-bis-acrylamide copolymer.

### DISCLOSURE OF THE INVENTION

The present invention, as defined in the claims, is aimed at the creation of a material which, on the one hand, is fairly resistant to degrading activity of enzymes, macrophages and phagocytes of the body, and on the other has adequate degree of resorption.

It is known that even negligible changes in the reagents ration during the synthesis of polyacrylamide gel lead to sharp changes in gel resorption speed inside the biological tissues (Doctoral thesis for PhD in Chemistry, Lopatin V.V. "Structure and properties of polyacrylamide gels in medicine", p.222).

In this respect the goal was to create a polyacrylamide-based material, the structure of which could allow for gradually changing the biodegradation time of the material by means of step-by-step alteration in the ratio of the reagents. During the synthesis, this would allow to predict the biodegradation time of implant in the body.

Another goal is to create the possibility for synovial fluid to enter the material when it is used in orthopedics for plastic joints.

Set goals were achieved by offering the material for medicinal purpose - a polyfunctional biocompatible hydrogel consisting of copolymer of acrylamide and N,N'-methylene-bis-acrylamide and water, wherein, according to the invention, the material additionally contains hyaluronic acid included into the structure with the following ratio of the components in mass%:
Acrylamide 0.9 - 8.2,
N,N'-methylene-bis-acrylamide 0.1 - 1.8,
Hyaluronic acid 0.1 - 2.0,
Water up to 100.0
As hyaluronic acid, the hydrogel primarily contains hyaluronic acid or its salt, for example, sodium salt with molecular weight 0.3 to 2.5 MDa. The hydrogel may also include silver ions in the amount of 0.0001-0.0025 mass%.

Set goals were also achieved by creating a manufacturing method of a polyfunctional biocompatible hydrogel for medicinal purposes, which included copolymerization of respective quantities of acrylamide and N,N'-methylene-bis-acrylamide in an aqueous medium in the presence of a peroxide polymerization activator. According to the invention, before polymerization, hyaluronic acid is added to the reaction mass of acrylamide and N,N'-methylene-bis-acrylamide, then the mass is aerated with inert gas for 5-15 min., then polymerization takes place at 69-74°C for 16-19 hours.
Primarily ammonium persulfate is used as the peroxide polymerization activator and argon gas is used as the inert gas.

An alternative method of manufacture of the polyfunctional biocompatible hydrogel for medicinal purposes is also provided herein, according to which hyaluronic acid hydrogel is mixed with suitable for medicinal use polyacrylamide gel to homogenous substance in an inert gas medium, such as argon, for example; said polyacrylamide is obtained by copolymerization of acrylamide and N,N'-methylene-bis-acrylamide in an aqueous disperse medium in the presence of mostly ammonium persulfate or hydrogen peroxide.

Depending on the viscosity of the starting gels, the process of mixing is done at the speed of 50 - 2500 r/min.

A manufacturing method of polyacrylamide gel suitable for preparation of the material is known and described, for example, in the patents RU 2127095 and RU 2127129.
In particular, it is possible to use ready polyacrylamide (containing acrylamide copolymer 0.9-8.2 mass% and 0.1 - 1.8 mass% of N,N'-methylene-bis-acrylamide and water) which is obtained according to method described in the patents RU 2127095 and RU 2127129.

It is also possible to use ready polyacrylamide (containing acrylamide copolymer 0.9-8.2 mass% and 0.1 - 1.8 mass% of N,N'-methylene-bis-acrylamide), which is obtained, for example, by copolymerization of the components in the presence of ammonium persulfate or hydrogen peroxide at 72±2°C for 18 hours.
In other particular cases, it is possible to use ready polyacrylamide gel (containing acrylamide copolymer 0.9-8.2 mass% and 0.1 - 1.8 mass% of N,N'-methylene-bis-acrylamide) suitable for medical applications for example as an implant for endoprosthetics of facial soft tissue, breast tissue, penis, calves, vocal cords and other tissues similar in density to gel; for application in urology and orthopedics.

To saturate the material with silver ions, water pre-saturated with silver ions, for example, by electrolysis is used.
Maximal limits of acrylamide, N,N'-methylene-bis-acrylamide and hyaluronic acid in the material are selected in the experiment for the purpose of achieving desired physico-mechanical characteristics.

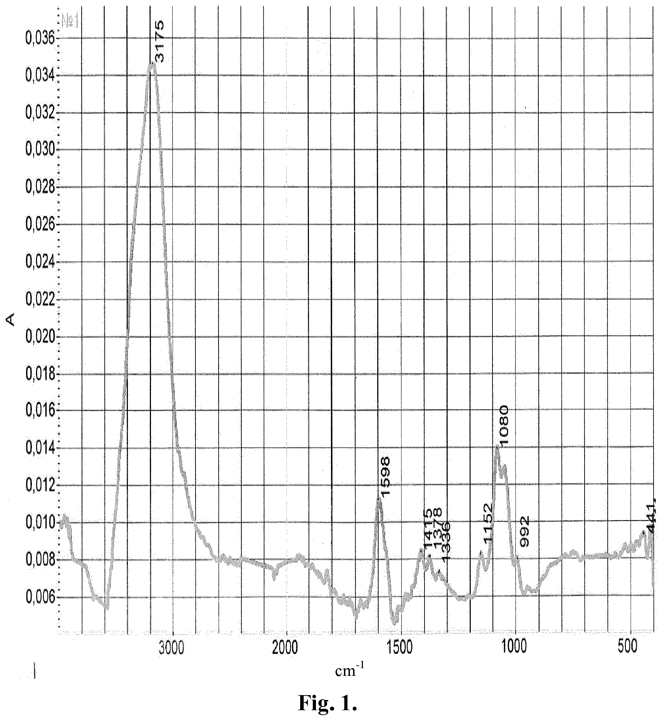

It was experimentally found that the described methods of manufacture of the material allow linear molecules of hyaluronic acid or its salts to be embedded into the interspatial slots of polyacrylamide hydrogel and therefore making physico-chemical links with it (Figs. 1-4 show IR-spectrum of samples).

The invention provides a material having positive properties of both polyacrylamide gels and hyaluronic acid gels. Moreover, the presence of hyaluronic acid molecules in the material allows synovial fluid to attach more easily to the material mesh and mix there with embedded hyaluronic acid. That leads to a prolonged treatment effect when the hydrogel is used in orthopedics.

Additionally, it was found that the hyaluronic acid in the material is presented in a stabilized state. Due to this, the sterilization of the finished product can be carried out at 120°C (see Examples of manufacturing methods of the material). However, it is known that hyaluronic acid is very heat-sensitive and boiling even for a short period of time results in irreversible changes of its properties (patent RU 2102400 published on 20.01.1998, "Temperature effect on dynamic rheological characteristics of hyaluronan", Hylana and Synvisc® (http://hyamatrix.ru/specialist/cosmetologists/hyaluronic_acid/vliyanie_temper atury_na_dinamicheskie_reologicheskie_osobennosti_gk_gilana_i_synvisc/).

### BRIEF DESCRIPTION OF THE FIGURES

For better understanding, examples of specific manufacturing methods of the novel biocompatible hydrogel are given with the reference to the illustrations.
Figs. 1-4 show IR-spectrums of the following compounds:
   Fig.1 shows an IR-spectrum of 1% solution of hyaluronic acid (HA) with molecular weight 2.5 MDa;

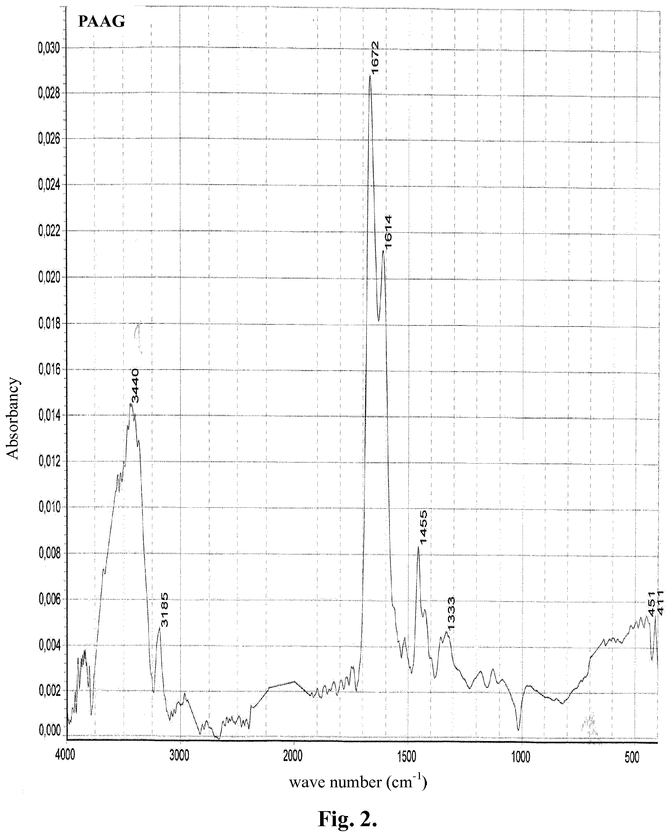

Fig.2 shows an IR-spectrum of a polyacrylamide sample (PAAG) containing in mass% 4.1 of acrylamide (AA), 0.1 of N,N'-methylene-bis-acrylamide (BAA) and the rest being water. The PAAG is obtained by copolymerization of the components in the presence of ammonium persulfate at the temperature of 72±2°C (measured with thermostat) for 18 hours;

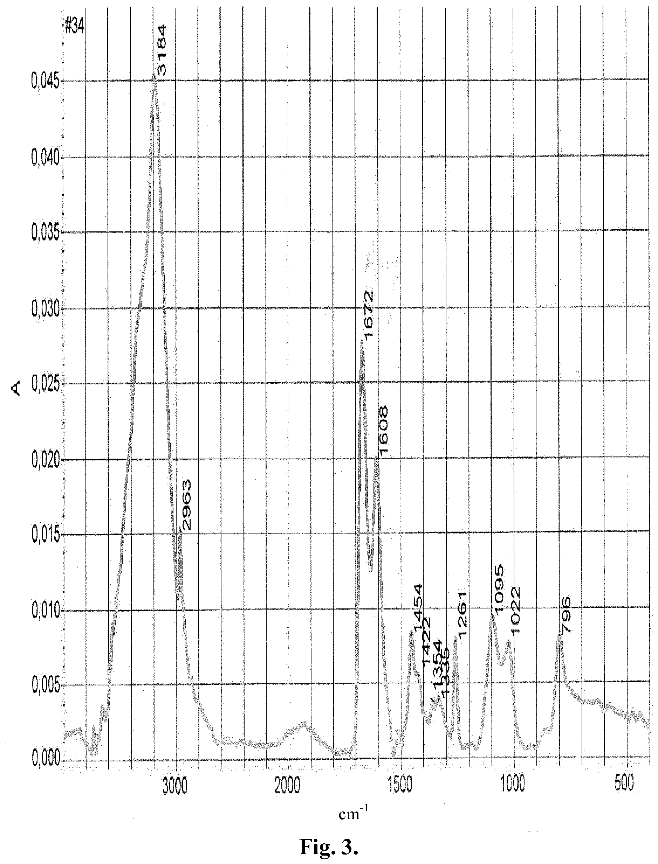

Fig.3 shows an IR-spectrum of the novel material in the form of a copolymer of hyaluronic acid (HA), acrylamide (AA) and N,N'-methylene-bis-acrylamide (BAA). The sample is obtained by copolymerization of the components at aeration of reaction mass with argon for 10 mins, followed by polymerization at 72±2°C (measured with a thermostat) for 18 hours. The sample contains, mass%: AA - 4.0%, BAA - 0.1%, HA - 0.1% and the rest is water;

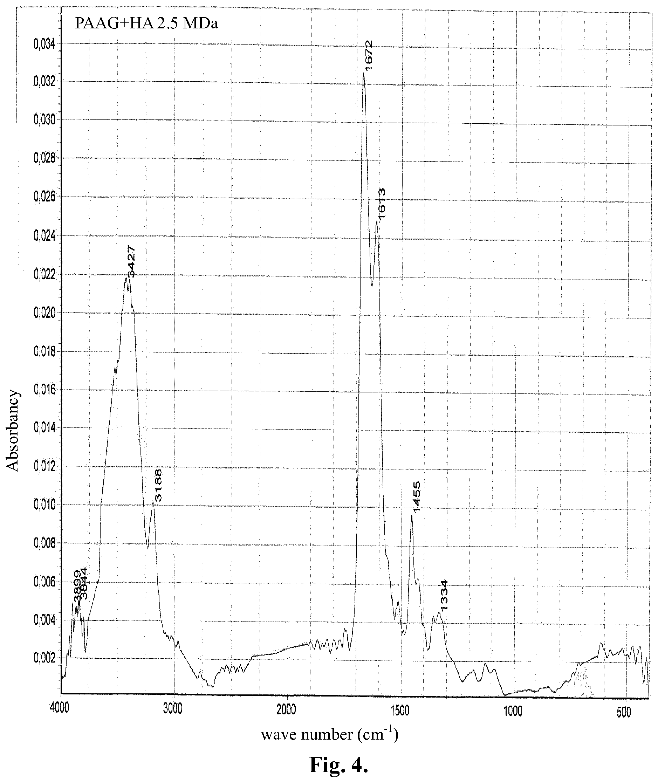

Fig. 4 shows an IR-spectrum of the sample of the novel material in the form of a composition obtained by mechanical mixing of 2% hyaluronic acid gel with ready polyacrylamide (PAAG) to the homogenous state. Ready PAAG contains, mass%: AA - 4.0%, BAA - 0.1%, the rest is water and obtained by polymerization of the components at 72±2°C for 18 hours. The sample of the invented material contains mass%: hyaluronic acid (HA) - 0.1%, acrylamide (AA) - 4.0%, N,N'-methylene-bis-acrylamide (BAA) - 0.1%, and water up to 100%.

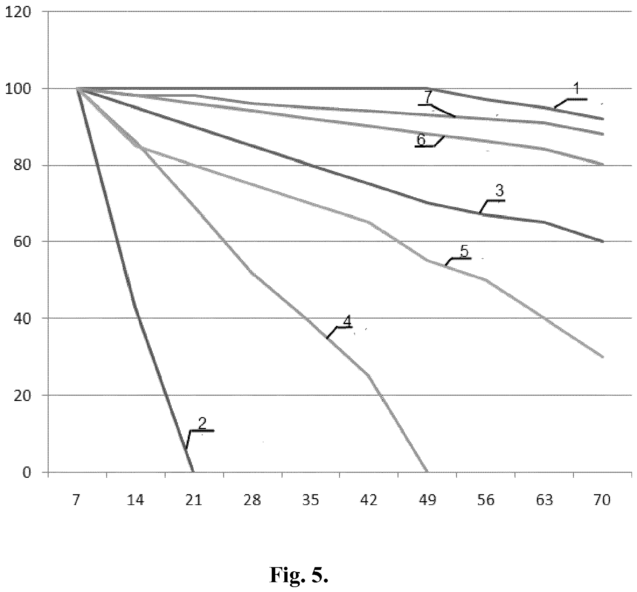

Fig.5 shows a resorption graph of samples of the novel material, hyaluronic acid and ready polyacrylamide (PAAG) samples where X-axis is time in days, Y-axis is volume of the material in % to the volume implanted:
   Curve 1 represents the PAAG (dry residue 4.2 mass%) containing acrylamide 4.1 mass%, N,N'-methylene-bis-acrylamide 0.1 mass% and the rest being water; obtained by polymerization of the components at 72±2°C for 18 hours.
   Curve 2 represents ready PAAG (dry residue 2 mass%) containing acrylamide 1.9 mass% , N,N'-methylene-bis-acrylamide 0.1 mass% and the rest being water; obtained by copolymerization of the components at 72±2°C for 18 hours.
   Curve 3 represents 2.5% hyaluronic acid (Mw 2.5 MDa) cross-linked with 1,4-butanediol diglycidyl ether (see patent RU 2382052 published 20.02.2010).
   Curve 4 represents 1% hyaluronic acid gel (Mw 2.5 MDa).
   Curve 5 represents 2.5% hyaluronic acid gel (Mw 2.5 MDa).
   Curve 6 represents the novel material which is a copolymer of acrylamide, N,N'-methylene-bis-acrylamide and hyaluronic acid. The sample is obtained by copolymerization of the components in the aqueous medium and aeration of reaction mass with argon for 10 mins, followed by polymerization at 72±2°C (measured with a thermostat) for 18 hours. The sample contains, mass%: hyaluronic acid - 0.3%, acrylamide - 4.1%, N,N'-methylene-bis-acrylamide - 0.1% and water up to 100%.
   Curve 7 represents the novel material obtained by mechanical mixing of hyaluronic acid gel with ready polyacrylamide gel (PAAG) to the homogenous substance, where the PAAG is obtained by copolymerization of the components at 72±2°C for 18 hours. The sample of novel material contains, mass% hyaluronic acid - 0.3%, acrylamide - 4.1%, N,N'-methylene-bis-acrylamide - 0.1% and water up to 100%.

At the presented spectrums of hyaluronic acid (Fig.1), it is seen that the highest point is located near 3175 cm⁻¹. This point corresponds to hydrogen bonds, which are pertinent to hydroxyl groups in hyaluronic acids. The broad line with the maximum near 3180 cm⁻¹ represents hydroxyl groups in hyaluronic acid bonded with hydrogen bonds.

In the spectrums of polyacrylamide (PAAG) samples (Fig.2), two intense lines 1670 cm⁻¹ and 1610 cm⁻¹ are seen, which are typical for fluctuation of amide groups Amid I and Amid II.
The maximum of 3440 cm⁻¹ is typical for -C(=O)-NH² in acrylamide.

As seen from the presented spectrums (Fig.3), in the novel material (hydrogel) in form of copolymer of acrylamide, N,N'-methylene-bis-acrylamide and hyaluronic acid the peaks are seen, which are typical for polyacrylamide as well as for hyaluronic acid.

The shifting of lines in the area of 3175 cm⁻¹ which are typical for hydrogen bonds of hydroxyl groups of hyaluronic acid to 3184 cm⁻¹ most likely are due to the fact that between hyaluronic acid and PAAG the coordinate chemical bonds are formed, but not the covalent chemical bonds. IR-spectrums of the material obtained by mechanical mixing of hyaluronic acid gel and the pre-processed polyacrylamide are shown in Fig. 4.

On this spectrum (Fig. 4), one sees peaks of 1614 cm⁻¹ and 1672 cm⁻¹ typical for polyacrylamide gels. The peak of 3175 cm⁻¹ characterizing hydrogen bonds of HA has moved to 3186 cm⁻¹. This is evidence of the creation of coordinate chemical bonds between HA and PAAG, because the samples of novel material obtained by different ways (copolymerization or mechanical mixing) have similar structures according to the received IR-spectrums. It is possible to assume that physico - mechanical characteristics, in particular viscosimetric properties of those samples, could be also similar.

However, viscosimetric properties of these samples have significant differences as shown in the examples, disclosing the invention.

### EXAMPLES OF THE INVENTION

To make the novel material take:
- acrylamide: C₃H₅NO, molecular weight 71.08, white crystal odorless powder; melting temperature 84.5°C; manufactured by Sigma (catalogue "Reagents for biochemistry and research in natural science" SIGMA, 1999, p.47, catalogue no. NºA8887);
- N,N'-methylene-bis-acrylamide: C₇H₁₀N₂O₂, molecular weight 154.16, white crystal odorless powder; melting temperature 185 °C, manufactured by Sigma (catalogue "Reagents for biochemistry and research in natural science" SIGMA, 1999, p.696, catalogue NºM7256);
- hyaluronic acid or its sodium salt with molecular weight 0.5 - 2.5 MDa. It is possible to use hyaluronic acid from microbiological sources;
- ammonium persulfate: (NH₄)₂S₂O₈° -molecular weight 228.19; colorless plate-like crystals; breaking temperature 120°C; manufactured by Sigma (catalogue "Reagents for biochemistry and research in natural science" SIGMA, 1999, p.117).

All above-mentioned monomers are suitable for biological purposes and do not require additional purification. The novel hydrogel may also include ions of silver produced by electrolysis.

Water shall be bidistilled and apyretic (pH 5.4 - 6.6).

A first method of manufacture generally is carried out as follows.

Take apyretic bidistilled water (pH 5.4-6.6). Portion of HA (Mw 0.5 - 2.5 MDa) is placed into the vessel with ¼ portion of total water and left to swell for 70-130 hours until a jelly homogenous mass is formed. Make portions of acrylamide and N,N'-methylene-bis-acrylamide in ratio 100:1 - 100:3 and ammonium persulfate in the amount of 0.6 -0.9 %. Portions of acrylamide, N,N'-methylene-bis-acrylamide and ammonium persulfate are diluted in apyretic bidistilled water (¾ of total water). When necessary, water with silver ions can be used. All weighed portions of ingredients are diluted in an argon gas medium. Prepared solutions are filtered and mixed with hyaluronic acid gel into the reaction mass. The reaction mass is aerated with argon for 5-15 mins and then it is polymerized at 69-74°C for 16-19 hours. The resulting material is packaged into the vessels or syringes of required volume and autoclave at 120°C and pressure 1.2 atm for 20 mins.

### Example 1: Manufacture of the novel material by copolymerization of hyaluronic acid with acrylamide and N,N'-methylene-bis-acrylamide (synthesis method)

To produce the hydrogel (samples Nº3 and Nº4), 300ml of purified apyretic bidistilled water (pH 5.4) are used. 0.1g of HA (Mw 2.5 MDa) are placed into 75 ml of water and left to swell for 72 hours in an argon gas medium. The remaining 225 ml of water is used in electrolysis to obtain water with silver ions with a concentration of 5mg/l.

8.7 g of acrylamide, 0.195 g N,N'-methylene-bis-acrylamide and 0.26 g of ammonium persulfate are diluted in 225ml of water with silver ions. Dilution of ingredients is also done in an argon medium. The obtained solution is filtered through a membrane filter (FMNC-8.0, manufactured by VLADISART, Russia) and mixed with hyaluronic acid hydrogel into a reaction mass. The reaction mass is aerated with argon gas for 5 mins. Polymerization is carried out in the thermostat at 72°C for 18 hours. The resulting material was packaged into the syringes of necessary volume and sterilized by autoclaving at 120°C and pressure 1.2 atm. for 20 mins. Samples 1, 2, 5, 6, 7, 8 and 9 (see Table 1) were prepared the same way. Specific quantities of the components for these samples, water pH, swelling time of HA, temperature and time of polymerization (in the table of thermosetting) are shown in Table 1.

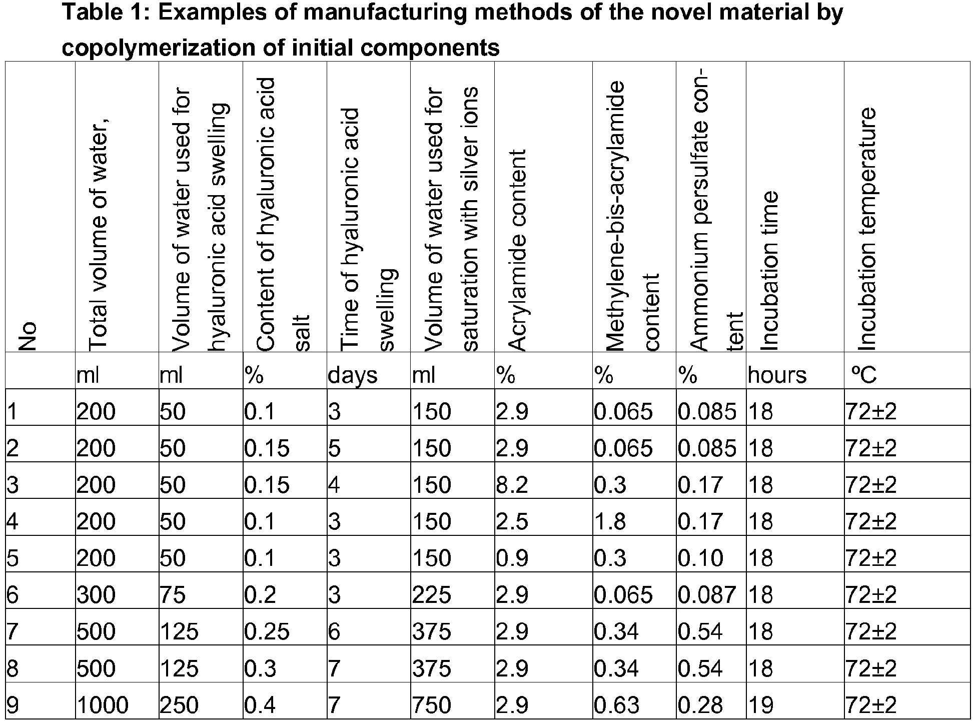

As the samples of the material obtained by copolymerization are not thick-flowing liquids, but elastic gel-like substances which, however, can be easily squeezed out through a needle, their viscosity properties could not be determined.

To characterize these systems the "shearing of elasticity" (G) parameter was used, which is measured by a spherical indenter penetration. The data on the characteristic properties depending on the composition of the gel are shown in Table 2.

**Table 2: The influence of the composition of the novel material obtained by joint copolymerization of the components on the modulus of elasticity, where AA is acrylamide, BAA is N,N'-methylene-bis-acrylamide, and HA is hyaluronic acid.**

| Sample No | AA and BAA content, mass% | Hyaluronic acid (HA) content, mass% | Dry residue, mass% | % HA to dry residue | % HA to polyacryla mide gel | Shearing of elasticity, G kPa |
|---|---|---|---|---|---|---|
| 1 | AA-0.9; BAA-0.3 | 2 | 3.2 | 62.5 | 2.0 | 0.4 |
| 2 | AA-0.9; BAA-0.3 | 0.1 | 1.3 | 7.69 | 0.1 | 0.7 |
| 3 | AA- 8.2; BAA - 0.3 | 2.0 | 10.5 | 10.05 | 19.05 | 1.4 |
| 4 | AA- 8.2; BAA - 0.3 | 0.1 | 8.6 | 1.16 | 0.1 | 1.6 |
| 5 | AA-0.9; BAA-2.5 | 2.0 | 5.4 | 37.4 | 2.0 | 0.6 |
| 6 | AA-0.9; BAA-2.5 | 0.1 | 3.5 | 2.86 | 2.86 | 3.2 |
| 7 | AA-4.0; BAA-1.5 | 1.0 | 6.0 | 16.6 | 1.0 | 1.6 |
| 8 | AA-4.0; BAA-1.5 | 0.5 | 6.0 | 8.33 | 0.5 | 2.0 |

### Example 2

### A manufacturing method for the novel material in the form of a composition of polyacrylamide gel with hyaluronic acid by mechanical mixing of HA hydrogel with ready polyacrylamide gel (PAAG), dry residue 4.3 mass%, containing AA 4.0 mass% and BAA 0.3 mass%, the rest being water, obtained by polymerization of the components at 72±2°C for 18 hours.

In a general way, the second embodiment of the method was carried out as follows:
Hyaluronic acid with molecular weight 0.5 ÷ 2.5 MDa, 1-2% concentration, was left to swell for 72-120 hours in an argon gas medium. The resulting hyaluronic acid hydrogel was combined with a ready polyacrylamide gel (PAAG), obtained by AA and BAA copolymerization in an aqueous disperse medium in the presence of ammonium persulfate or hydrogen peroxide, with the reaction mass incubated at the temperature of 72±2oC for 18 hours.

Then the hydrogel of hyaluronic acid (HA, the specific quantity which is specified in Table 3) was placed in a vessel for mixing where a certain quantity (also specified in Table 3) of polyacrylamide gel was added. Then the content was stirred with a mechanical overhead stirrer at the speed of 500 r/min to the homogeneous state. The stirring was carried out at different ratios of the ready PAAG and hyaluronic acid hydrogels. The obtained material was packaged into vessels or syringes of required volume and autoclaved at 120°C and pressure 1.2 atm for 20 mins.
The data on the ratio of the components and viscous properties of the obtained material are presented in Table 3.

**Table 3: Viscosimetric properties of the offered material in the form of the composition obtained by mixing pre-processed PAAG with hyaluronic acid**

| Sample No | Ratio of components (mass%) | | | Viscosity, Pa |
|---|---|---|---|---|
| | PAAG, containing AA-4.1 mass%, BAA-0.2 mass%, g | HA - 1 1% hydrogel, g | HA - 2 2% hydrogel, g | |
| 1 | 5 | 95 | - | 2.6 x 10⁻³ |
| 2 | 50 | 50 | - | 4.2 |
| 3 | 95 | 5 | - | 3.9 |
| 4 | 5 | - | 95 | 1.8 x 10⁻³ |
| 5 | 50 | - | 50 | 3.8 |
| 6 | 95 | - | 5 | 3.5 |

As shown by the data presented in Tables 2 and 3, the viscosimetric properties of the samples of the material obtained by copolymerization of hyaluronic acid with acrylamide and methylene-bis-acrylamide, and of the samples of the material obtained by mechanical mixing of hyaluronic acid hydrogel and a ready polyacrylamide gel (PAAG) are more than 1000 times different. This difference is accounted for by the method of manufacturing of the new material. The material in the form of a hydrogel obtained by copolymerization of acrylamide, methylen-bis-acrylamide and hyaluronic acid has a cross-linked polyacrylamide structure, embedded with molecules of hyaluronic acid. The hydrogels obtained by mechanical mixing of ready PAAG with hyaluronic acid are a mechanical link of polyacrylamide gel mesh fragments in the hyaluronic acid hydrogel.

### Experiments on rats

To study the resorption speed of the novel material depending on the composition and method of manufacturing thereof, an experimental study of tissue reaction and resorption speed at subcutaneous administration of different samples of the offered material obtained by different variants of the method was carried out.

### Experimental procedure

White laboratory rats - males, body weight 150-180 g, anesthetized with "Zometa - Rometor" combination were injected subcutaneously in the scapular region on both sides of the midline with 1.5 ml of the hydrogels studied. Experimental animals were withdrawn from the study on day 3, 7, 21, 35, 42, 49, 56, 63 and 70. Encapsulated gel implants with surrounding tissue were sampled from the site of administration. The material was fixed in 10% formalin solution and embedded in paraffin. Paraffin sections were stained with hematoxylin and eosin. The specimens were viewed under the light microscope BX-51. The experimental results are presented as a graph in Figure 5, where X-axis is the time of animal withdrawal from the experiment in days and Y-axis is the volume of the material in % to the volume implanted.

As can be seen from the materials presented (Fig. 5), by means of changing the ratio of PAAG and HA in the offered material, as well as the method for the production thereof, a hydrogel with predicted resorption rate can be obtained.

Toxicological studies of the samples of the novel material in the form of a hydrogel named "Matrexsyn" were carried out in accordance with the Standards Series GOST R ISO 10993 (GOST R ISO 10993-1-2009 - GOST R ISO 10993-11-2009) "Biological evaluation of medical devices". Toxicological tests showed that aqueous extracts from the samples of the novel hydrogel produced no hemolytic effect in experiments "in vitro" with isolated erythrocytes of rabbits. A complete absence of hemolytic activity was established, an acceptable value being 2%.
In the acute toxicity experiment on white mice upon parenteral administration of the hydrogel samples at a dose of 50.0 ml per 1 kg of body weight, no animal deaths or clinical signs of intoxication occurred. The general condition of the experimental mice, their behavior, feed intake, coat condition did not differ from those of the controls.
The experimental mice autopsy established that the tissues at the site of the hydrogel administration, regional lymph nodes, internal organs (liver, kidney, spleen) were within the physiological range of the controls. There were no statistically significant differences in body weight dynamics, clinical and biochemical blood counts, internal organs mass coefficients in the experimental animals as compared to controls after subcutaneous implantation of the gel.

### Industrial applicability

Thus, the given examples of the particular embodiment of the above show that the novel material can be obtained by the proposed variants of the method that ensure obtaining a material with a predictable resorption rate and time after the implantation thereof into an animal or human body. The novel material does not virtually induce tissue reaction, does not cause sensitization, does not cause dystrophic or necrotic changes and can be used for implantation into an animal or human body.

The novel material compared with the prior art polyacrylamide hydrogel "Argiform" (TU 9398-002-52820385-2008) produced under the trademark Noltrex™, having the following composition: 3-dimensional polyacrylamide - 4.5 ± 1.5%, bi-distilled water 95.5±1.5%, silver ions - 0.01 -0.02% (http://www.rlsnet.ru/pcr tn id 34552.htm), has a predictable resorption rate. Compared with another known synovial fluid substitute, Synocrom® (a synovial fluid prosthesis) containing sodium hyaluronate with a molecular weight of about 1.6 MDa, auxiliaries and water for injection (http://slovari.yandex.ru/∼ 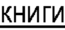 / 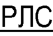 / 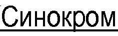 %20 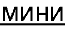 %20 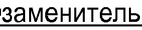 %2 0 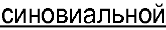 %20 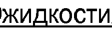 /), the offered biocompatible material remains in the joint for at least 6 months. The residence time of the material is predicted based on the composition and method of manufacturing thereof. Furthermore, in contrast to the known drugs based on reticulated or non-reticulated hyaluronic acid or its salts, the novel material can be stored at room and higher temperatures and sterilized at 120°C, which increases the safety of the material to the recipient.

## Claims

1. A material for medical purposes based on a polyacrylamide hydrogel, comprising a copolymer of acrylamide and N,N'-methylene-bis-acrylamide and water, ***characterized in tha**t* it additionally comprises hyaluronic acid included into the structure of the hydrogel, with the following ratio of the components in mass%:
| | |
|---|---|
| Acrylamide | 0.9 - 8.2, |
| N,N'-methylene-bis-acrylamide | 0.1 - 1.8, |
| Hyaluronic acid | 0.1 - 2.0, |
| Water | up to 100.0 |

2. The material of claim 1, ***characterized in that*** it contains as hyaluronic acid the hyaluronic acid itself or its salt, with molecular weight of 1.5-2.5 MDa.

3. The material of claim 1, ***characterized in that*** it additionally comprises silver ions in the amount of 0.0001 -0.0025 mass%.

4. A method for production of the material for medical purposes of claim 1 by copolymerization of acrylamide and N,N'-methylene-bis-acrylamide in aqueous medium in the presence of peroxide polymerization activator, ***characterized in that*** before polymerization hyaluronic acid is added to the reaction mass, then the mass is aerated with inert gas for 5 - 15 min, then polymerization is conducted at 69 - 74°C for 16 - 19 hours.

5. The method of claim 4, ***characterized in that*** as peroxide polymerization activator primarily ammonium persulfate is used.

6. The method of claim 4, ***characterized in that*** as inert gas argon gas is used.

7. The method of claim 4, ***characterized in that*** as water the water pre-saturated with silver ions, for example, by electrolysis is used.

8. A method of production of the material for medical purposes of claim 1, consisting in that a hyaluronic acid hydrogel is mixed with suitable for medicinal use polyacrylamide gel containing acrylamide and N,N'-methylene-bis-acrylamide to a homogenous substance in an inert gas medium.

9. The method of claim 8, ***characterized in that*** as polyacrylamide hydrogel a gel obtained by copolymerization of appropriate quantities of acrylamide and N,N'-methylene-bis-acrylamide in aqueous disperse medium in the presence of peroxide polymerization activator, mostly ammonium persulfate or hydrogen peroxide, is used.

10. The method of claim 8, ***characterized in that*** as inert gas argon is used.

11. The method of claim 8, ***characterized in that*** as water the water pre-saturated with silver ions, for example, by electrolysis is used.

## Patentansprüche

1. Material für medizinische Zwecke, basierend auf einem Polyacrylamidhydrogel, umfassend ein Copolymer aus Acrylamid und N,N'-Methylen-bis-acrylamid und Wasser, ***dadurch gekennzeichnet, dass*** es zusätzlich Hyaluronsäure umfasst, die in der Struktur des Hydrogels eingeschlossen ist, mit dem folgenden Verhältnis der Komponenten in Massen-%:
| | |
|---|---|
| Acrylamid | 0,9 - 8,2, |
| N,N'-Methylen-bis-acrylamid | 0,1 - 1,8, |
| Hyaluronsäure | 0,1 - 2,0, |
| Wasser | bis auf 100,0 |

2. Material nach Anspruch 1, ***dadurch gekennzeichnet, dass*** es als Hyaluronsäure die Hyaluronsäure selbst oder ihre Salze, mit einem Molekulargewicht von 1,5-2,5 MDa enthält.

3. Material nach Anspruch 1, ***dadurch gekennzeichnet, dass*** es zusätzlich Silberionen in einer Mange von 0,0001 - 0,0025 Massen-% enthält.

4. Verfahren zur Herstellung des Materials für medizinische Zwecke nach Anspruch 1 durch Copolymerisation von Acrylamid und N,N'-Methylen-bis-acrylamid in wässrigem Medium in Anwesenheit von Peroxidpolymerisationsaktivator, ***dadurch gekennzeichnet, dass*** vor der Polymerisation Hyaluronsäure zu der Reaktionsmasse hinzugefügt wird, dann die Masse mit Inertgas 5 - 15 min lang belüftet wird, dann die Polymerisation bei 69 - 74 °C 16 - 19 Stunden lang durchgeführt wird.

5. Verfahren, nach Anspruch 4, ***dadurch gekennzeichnet, dass*** als Peroxidpolymerisationsaktivator vorrangig Ammoniumpersulfat verwendet wird.

6. Verfahren, nach Anspruch 4, ***dadurch gekennzeichnet, dass*** als Inertgas Argongas verwendet wird.

7. Verfahren, nach Anspruch 4, ***dadurch gekennzeichnet, dass*** als Wasser das Wasser verwendet wird, das mit Silberionen vorgesättigt ist, beispielsweise durch Elektrolyse.

8. Verfahren zur Herstellung des Materials für medizinische Zwecke nach Anspruch 1, daraus bestehend, dass ein Hyaluronsäurehydrogel mit für medizinische Zwecke geeignetem Hydrogel, welches Acrylamid und N,N'-Methylen-bis-acrylamid enthält, zu einer homogenen Substanz in einem Inertgasmedium gemischt wird.

9. Verfahren nach Anspruch 8, ***dadurch gekennzeichnet, dass*** als Polyacrylamidhydrogel ein Gel verwendet wird, das erhalten wurde durch Copolymerisation geeigneter Mengen Acrylamid und N,N'-Methylen-bis-acrylamid in wässrigem Dispergiermedium in Anwesenheit von Peroxidpolymerisationsaktivator, wobei zumeist Ammoniumpersulfat oder Wasserstoffperoxid verwendet wird.

10. Verfahren, nach Anspruch 8, ***dadurch gekennzeichnet, dass*** als Inertgas Argon verwendet wird.

11. Verfahren, nach Anspruch 8, ***dadurch gekennzeichnet, dass*** als Wasser das Wasser verwendet wird, das mit Silberionen vorgesättigt ist, beispielsweise durch Elektrolyse.

## Revendications

1. Matériau à usage médical à base d'hydrogel de polyacrylamide, comprenant un copolymère d'acrylamide et de N,N'-méthylène-bis-acrylamide et de l'eau, **caractérisé en ce qu'**il comprend en outre de l'acide hyaluronique inclus dans la structure de l'hydrogel, avec le rapport suivant des constituants en % en masse :
| | |
|---|---|
| Acrylamide | 0,9 à 8,2, |
| N,N'-méthylène-bis-acrylamide | 0,1 à 1,8, |
| Acide hyaluronique | 0,1 à 2,0, |
| Eau | jusqu'à 100,0 |

2. Matériau selon la revendication 1, **caractérisé en ce qu'**il contient comme acide hyaluronique l'acide hyaluronique lui-même ou son sel, avec un poids moléculaire de 1,5 à 2,5 MDa.

3. Matériau selon la revendication 1, **caractérisé en ce qu'**il comprend en outre des ions argent à raison de 0,0001 à 0,0025 % en masse.

4. Procédé de production du matériau à usage médical selon la revendication 1 par copolymérisation d'acrylamide et de N,N'-méthylène-bis-acrylamide en milieu aqueux en présence d'activateur de polymérisation peroxyde, **caractérisé en ce qu'**avant la polymérisation, de l'acide hyaluronique est ajouté à la masse réactionnelle, puis la masse est aérée avec du gaz inerte pendant 5 à 15 minutes, puis la polymérisation est conduite à 69 à 74 °C pendant 16 à 19 heures.

5. Procédé selon la revendication 4, **caractérisé en ce que** l'on utilise comme activateur de polymérisation peroxyde principalement le persulfate d'ammonium.

6. Procédé selon la revendication 4, **caractérisé en ce que** l'on utilise comme gaz inerte l'argon gazeux.

7. Procédé selon la revendication 4, **caractérisé en ce que** l'on utilise comme eau une eau pré-saturée avec des ions argent, par exemple, par électrolyse.

8. Procédé de production du matériau à des fins médicales selon la revendication 1, consistant en ce qu'un hydrogel d'acide hyaluronique est mélangé avec un gel de polyacrylamide approprié pour un usage médicinal contenant de l'acrylamide et du N,N'-méthylène-bis-acrylamide jusqu'à substance homogène dans un milieu de gaz inerte.

9. Procédé selon la revendication 8, **caractérisé en ce que** l'on utilise, comme hydrogel de polyacrylamide, un gel obtenu par copolymérisation de quantités appropriées d'acrylamide et de N,N'-méthylène-bis-acrylamide dans un milieu de dispersion aqueux en présence d'activateur de polymérisation peroxyde, du persulfate d'ammonium ou du peroxyde d'hydrogène majoritairement.

10. Procédé selon la revendication 8, **caractérisé en ce que** l'on utilise comme gaz inerte de l'argon.

11. Procédé selon la revendication 8, **caractérisé en ce que** l'on utilise comme eau l'eau préalablement saturée en ions argent, par exemple par électrolyse.
